**Europäisches Patentamt**

**European Patent Office**

**Office européen des brevets**

(19)

(11) Veröffentlichungsnummer: **0 346 469**
A1

# EUROPÄISCHE PATENTANMELDUNG

(12)

Veröffentlicht nach Art. 158 Abs. 3 EPÜ

(21) Anmeldenummer: **88901160.7**

(22) Anmeldetag: **16.11.87**

Daten der zugrundeliegenden internationalen Anmeldung:

(86) Internationale Anmeldenummer:
**PCT/SU87/00130**

(87) Internationale Veröffentlichungsnummer:
**WO89/04638 (01.06.89 89/12)**

(51) Int.Cl.³: **A 61 B 17/34**

(43) Veröffentlichungstag der Anmeldung:
**20.12.89 Patentblatt 89/51**

(84) Benannte Vertragsstaaten:
**AT BE CH DE FR GB IT LI SE**

(71) Anmelder: **UNIVERSITET DRUZHGBY NARODOV IMENI PATRISA LUMUMBY**
**ul, Miklukho-Maklaya 6**
**Moscow, 117198(SU)**

(72) Erfinder: **KULIK, Very Pavlovich**
**Bolshoi Savvinsky per., 19-55**
**Moscow, 119435(SU)**

(72) Erfinder: **NOVIKOV, Valery Konstantinovich**
**ul. Vorovskogo, 18/9-38**
**Moscow, 121069(SU)**

(74) Vertreter: **Patentanwälte Beetz sen. - Beetz jun. Timpe - Siegfried - Schmitt-Fumian- Mayr**
**Steinsdorfstrasse 10**
**D-8000 München 22(DE)**

(54) **TROKAR.**

(57) Trokar, enthaltend eine hohle Hülse (1), in der ein Stilett (2) angeordnet ist, das einen durchgehenden Längskanal (4) aufweist und ein abgespitztes Ende (3) hat, das mindestens drei Flächen besitzt.

FIG. 1

## TROKAR

### Technisches Gebiet

Die Erfindung bezieht sich auf die medizinische Technik, insbesondere betrifft sie Einrichtungen zur Punktion und Stoffeinführung und genauer auf Trokare, und kann beim Durchstechen von natürlichen und pathologischen Leibeshöhlen, Blutgefäßen, Rückenmarkkanälen, bei subkutaner, intramuskulärar, intraorganer und Höhlenimplantation von Zellkulturen sowie Gewebe- und Organstücken, das heißt in der Chirurgie, Endokrinologie, Transplantologie, Neurologie und bei Durchführung endoskopischer Untersuchungen sowohl in der Medizin als auch in der Tierheilkunde Anwendung finden.

### Zugrundeliegender Stand der Technik

Zur Zeit hat sich wesentlich der Bereich der Anzeigen kleiner chirurgischer Eingriffe erweitert, bei denen man anstatt einer weiten Öffnung von Höhlen des Organismus, sowohl der natürlichen wie Brust- und Bauchhöhlen als auch der künstlichen wie ein Abszeß und andere, auf deren Durchstechen und die Einfürhung eines Instrumentes für eine Übersicht, Probeentnahme oder eine Behandlung wie Entleerung und Einführung von Heilmitteln sich beschränken kann. Darüber hinaus erschienen neue Operationen, solche wie eine Katheterisation von Gefäßen, endoskopische Untersuchungen, eine Implantation von biologischen Stoffen, und zwar Zellkulturen, Gewebe- und Organstücken, die einen Durchstich von Leibesgeweben und -organen erforderlich machen.

Dadurch sind die Anforderungen an Instrumente und Geräte, die eine Verwirklichung solcher Operationen möglich machen, insbesondere Anforderungen ihrer maximalen Sicherheit und eines minimalen Traumatismus für kranke Menschen und Tiere höher geworden. Von großer Bedeutung ist auch die Frage einer vollständigen Anästhesie des Durchstiches.

Allgemein bekannt ist, beispielsweise eine Punktionskanüle, dessen ein Ende schräg abgeschnitten ist, was ihm eine Abspitzung verleiht, und das andere Ende mit einer Vorrichtung zur Verbindung mit einer Spritze oder einem anderen Injektionssystem versehen ist. Der Kanal solch einer Kanüle kann mit einem abnehmbaren Mandrin obturiert werden, der sowohl zur Reinigung des Kanals als auch zur Verschließung desselben beim Durchstechen verwendet wird, damit das Blut oder das Gewebe in den Kanal nicht geraten und ihn nicht verstopfen. Injektions- und Punktionskanülen verwendet man zur Einführung von Heilmitteln, Blut und Blutersatzpräparaten intrakutan, subkutan, intramuskulär, intravenös, intraossär, zur Entleerung von mit biologischen Flüssigkeiten wie Exsudat, Blut, Eiter und anderengefüllten natürlichen und pathologischen Leibeshöhlen und zur Punktion von Rückenmarkkanälen sowohl in der Medizin (s. das Buch von G.E. Ostroverkhov, D.N. Lubottsky, Ju.M. Bomash "Operativnaya khirurgia i topograficheskaya anatomia", Moskau, 1972, Verlag "Meditsina", S. 38, 375) als auch in der Tierheilkunde (I.I. Magda "Operativnaya khirurgia s osnovami topograficheskoi anatomii domashnikh zhivotnykh", Moskau, 1970, Verlag "Kolos", S.59). Aber die Anwendung der Kanülen ist mit der Gefahr des Durchstechens von Blutgefäßen, Nerven, Rückenmark und besonders Innenorganen verbunden, wodurch lebensgefährliche Komplikationen wie Peritonitis, Pleuritis, Mediastinit, innere Blutungen, Neuritis, Lähmungen usw. entstehen. Außerdem kann der Durchmesser von Kanülen eine Größe von 2 bis 3 mm nicht überschreiten, wodurch ihre Anwendung, insbesondere zur Einführung von Gewebe- und Organstücken, zur Evakuierung eines Hämatominhaltes, Blutgerinnsels und Fibrins beschränkt wird. Durch Kanülen kann man, beispielsweise kein Endoskoprohr einführen. Schließlich ist es bei der Punktion einer Brust- und Bauchhöhle erforderlich, daß in diesen Höhlen Exsudat (Flüssigkeit) enthalten ist, sonst sei ein Durchstich unvermeidlich.

Eine der Beschränkungen der Injektions- und Punktionskanülen, und zwar ihr kleiner Durchmesser, ist in einem allgemein bekannten Trockar beseitigt.

Der Trokar besteht aus zwei Teilen: einem Stilett und einer Hülse, in der das Stilett untergebracht ist. Das eine Ende des Stiletts ist in Form einer drei- oder vierflächigen Pyramide abgespitzt, das andere Ende ist erweitert und stützt sich auf den Rand der Hülse. Der Trockar funktioniert wie folgt. Nach der Durchführung einer Lokalanästhesie und Ausführung eines kleinen Einschnitts in die Haut stellt der Chirurg das Instrument, indem er den zusammengesetzten Trokar in der rechten Hand hält und die Haut mit der linken Hand spannt, normal zur Oberfläche der Durchstichzone, schiebt den Trokar im Gewebe vor und durchsticht die Höhlenwand, danach zieht er das Stilett aus der Hülse heraus, durch die dann die Entleerung der Höhle und/oder die Einführung von Heilmitteln in diese vorgenommen wird (s. das Buch von G.E. Ostroverkhov, D.N. Lubotsky, Ju.M. Bomash "Operativnaya khirurgia i topograficheskaya anatomia", Moskau, 1972, Verlag "Meditsina", S. 574). In der Tierheilkunde wird der Trokar auch für das Durchstechen eines Magens, einer Harnblase und Vene angewandt (I.I. Mgda "Operativnaya khirurgia s osnovami topograficheskoi anatomii domashnikh zhivotnykh", Moskau, 1970, Verlag "Kolos", S. 222).

Eine Hauptgefahr der Verwendung des obenerwähnten Trokars besteht darin, daß die Innenorgane traumiert werden können, weil der Aufbau der Spitze des Stiletts die Neigung der Flächen zur Achse des Instrumentes unter einem Winkel von höchstens $30^o$ vorsieht. Außerdem werden bei der Anwendung solch eines Trokars eine Anästhesie und ein Durchstich getrennt durchgeführt, die Anästhesie eines Pleura- und Bauchfellblattes aber wird in der Regel wie oben erwähnt nicht durchgeführt, da eine Gefahr des Durchstechens der Innenorgane besteht, und deren Durchstich vom Trokar kränklich ist: nach dieser Schmerzhaftigkeit beurteilt man sogar das Durchgehen des Instrumentes durch sie. Dasselbe bezieht sich auch auf die Hirnhäute bei einer Rückenmarkpunktion. Schließlich kann der Trokar des üblichen Aufbaues ebenso wie Punktionskanülen nicht für die Punktion einer keine Flüssigkeit enthaltenden Höhle verwendet werden.

## Offenbarung der Erfindung

Die Aufgabe der vorliegenden Erfindung besteht in der Entwicklung solch eines Trokars, bei dem die Veränderung der Konstruktion seines Stiletts es ermöglicht, ihn weniger traumatisch zu machen und sein Anwendungsgebiet bedeutend zu erweitern.

Diese Aufgabe wird dadurch gelöst, daß im Trokar, der ein Stilett mit einem abgespitzten Ende und eine Hülse aufweist, in der das Stilett untergebracht ist, erfindungsgemäß, das Stilett einen Durchgangskanal hat und dessen abgespitztes Ende mindestens drei Flächen aufweist.

Das ermöglicht es, Verletzungen bei der Anwendung des Instrumentes geringer zu machen und gleichzeitig mit dem Durchstich der Anästhesie von Geweben, durch welche der Trokar durchgeht, zu verwirklichen, wodurch sein Anwendungsgebiet bedeutend erweitert wird.

Es ist zweckmäßig, daß der Schärfungswinkel der Schneidflächen des abgespitzten Endes des Stiletts zu einer Achse von 45 bis 90° beträgt.

Das ermöglicht das Spreizen von Geweben in Richtung des Vorschubs des Trokars, vermindert die Gefahr eines zufälligen Durchstechens von Blutgefäßen, Nerven und Innenorganen und somit erhöht die Zuverlässigkeit des Instrumentes.

Es ist wünschenswert, daß die Schneidflächen, die eine Abspitzung des Stiletts bilden, erfindungsgemäß, Schneidkanten haben. Das trägt zum leichteren Vorschub des Trokars im Gewebe verschiedener Dichte und Struktur bei.

Es ist auch zweckmäßig, daß das eine Ende des durchgehenden Kanals des Stiletts des Trokars, erfindungsgemäß, mindestens einen Austritt in unmittelbarer Nähe der Spitze zwischen benachbarten Flächen aufweist und das andere Ende des Kanals entsprechend den geometrischen Maßen der in diesen einzusetzenden Injektionssysteme erweitert ausgeführt ist.

Dadurch wird ermöglicht, den Trokar durch das Ende des Stiletts mit einer Spritze oder einem Infusionssystem vom anderen Typ, die eine Anästhesielösung enthaten, zu verbinden und die Anästhesie von Geweben, durch die das Instrument eingeführt wird, zu verwirklichen. Die Anordnung der Austrittsöffnungen des Kanals in der Nähe der Spitze gibt außerdem die Möglichkeit, eine Anästhesielösung vor dem Instrument einzuspritzen und nicht nur die Anästhesie sondern auch die hydraulische Präparation von Geweben durchzuführen, wodurch die Gefahr der Verletzung von Gefäßen, Nerven und Innenorganen noch mehr vermindert wird. Die kleinen Maße der Öffnungen des Kanals und ihre Anordnung zwischen den benachbarten Flächen der Abspitzung schließen praktisch die Möglichkeit aus, daß eine Verunreinigung, Verstopfung desselben mit Stoffen und Geweben, durch die der Trokar durchgeht, stattfinden.

Es ist wünschenswert, den Trokar mit einem abnehmbaren Hülsengriff zu versehen, dessen geometrische Maße den geometrischen Maßen des Behälters der Injektionssystems entsprechen.

Das ermöglicht es, eine Spritze und ein beliebiges Injektionssystem gegen die Druckkraft der Hand des Chirugen während des Vorschubs des Trokars im Gewebe zu schützen, und es dient auch als Prophylaxe eines Traumas des Chirurgen, wenn die Spritze zerdrückt wird, und auch als Prophylaxe aller anderen eventuellen Komplizierungen von der Seite des Injektionssystems, beispielsweise im Falle der Unterbrechung der Flüssigkeitszufuhr.

Kurzbeschreibung der Zeichnungen

Nachstehend wird die Erfindung durch eine eingehende Beschreibung dessen Ausführungsbeispiels und an Hand der beigelegten Zeichnungen näher erläutert, wobei es zeigen:

Fig. 1 eine Gesamtansicht des erfindungsgemäßen Trokars, zusammengesetzt mit einer Spritze (Längsschnitt);

Fig. 2 eine Gesamtansicht des erfindungsgemäßen Trokarstiletts, teilweise geschnitten;

Fig. 3 den Längsschnitt des gespitzten Endes des erfindungsgemäßen Trokarstillets;

Fig. 4 den Querschnitt des gespitzten Endes des erfindungsgemäßen Trokarstiletts durch die Ebene des Endes des Innenkanals desselben.

## Beste Ausführungsvariante der Erfindung

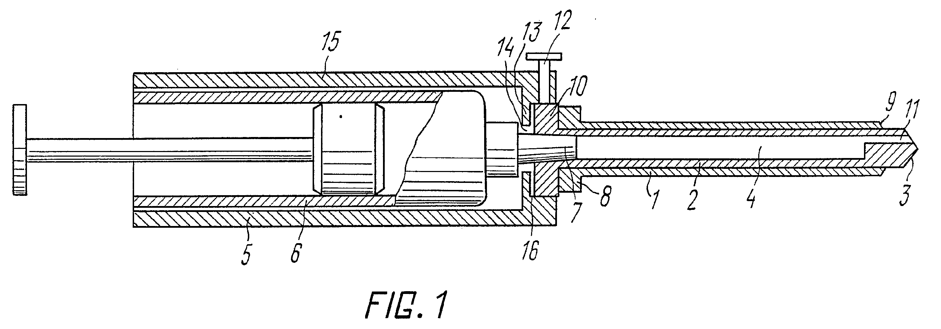

Der erfindungsgemäße Trokar sieht eine hohle Hülse 1, ein Stilett 2, das ein abgespitztes Ende 3 und einen durchgehenden Längskanal 4 aufweist, und einen Hülsengriff 5 (Fig.1) vor.

Die Hülse 1 wird auf das abgespitzte Stilett 2 aufgesetzt, an das der Hülsengriff 5 angeschlossen wird. In den Hülsengriff 5 wird ein Injektionssystem, beispielsweise eine Spritze 6 oder eine Gummiröhre mit dem Übergangsstück von einem System der Flüssigkeitsübertragung (in der Zeichnung nicht angedeutet), das in den erweiterten Endteil 7 des Kanals 4 des Stiletts 2 eingeht, eingesetzt. Das abgespitzte Ende 3 des Stiletts 2 ragt aus der Hülse 1.

Die Hülse 1 stellt einen hohlen Zylinder mit einem verdickten Ende 8 in Form eines Torus dar. Der Innendurchmesser der Hülse 1 ist über die ganze lange gleich und entspricht dem Außendurchmesser des Stiletts 2. Der Rand des anderen Endes 9 der Hülse 1 ist abgeschrägt.

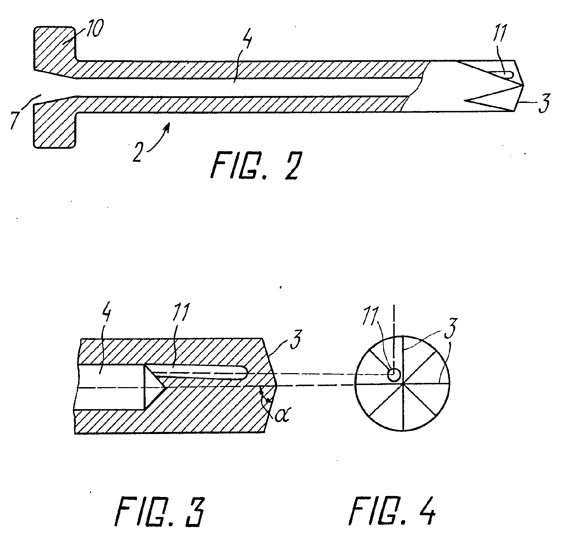

Das Stilett 2 (Fig. 2) stellt ein Zylinder mit einem Innenkanal 4, mit einem abgespitzten Ende 3 und mit einem verdickten Ende 10, das in Form eines Torus ausgeführt ist und zur Begrenzung der Bewegung des Stiletts 2 innerhalb des Kanals der Hülse 1 dient, dar.

Das abgespitzte Ende 3 des Stiletts 2 hat mindestens drei Flächen, aber im nachstehenden Beispiel ist es der Einfachheit halber kreuzartig abgebildet. Der Winkel α der Flächen des abgespitzten Endes 3 zur Achse des Stiletts 2 gleicht von 45 bis 90° (Fig. 3). Wenn die Schneiden der Flächen des Stiletts zu dessen Achse unter einem Winkel weniger als 45° abgeschräft sind, wird das abgespitzte Ende 3 des Stiletts zu scharf und erhöht sich die Gefahr des Durchstichs von Geweben anstatt einer Spreizung. Wenn der Schärfungswinkel der Schneiden der Flächen mehr als 90° ist, so wird das Ende 3 des Instrumentes in der Mitte nicht mehr abgespitzt: es wird entweder platt (bei 90°) oder mitten eingedrückt. Die Schneiden des abgespitzten Endes stellen die Schneidkanten dar. Der Kanal 4 des Stiletts 2 weist ein sich erweiterndes Ende 7 zur Verbindung mit einem Injektionssystem und mindestens ein engeres Ende 11 auf, das in der Nähe des abgespitzten Teils des Stiletts zwischen den Flächen zweier Nachbarschneiden mündet (siehe auch Fig. 3, 4).

Der Hülsengriff 5 (Fig. 1) ist ein Hohlzylinder, von dem das eine mit einer Schraube 12 zur Fixierung des verdickten Endes 10 des Stiletts 2 und einem Begrenzer 13 versehen ist, auf den sich die Spritze 6 oder ein beliebiges anderes Ende eines Injektionssystems stützt, wenn dessen Durchmesser größer als der Durchmesser des verdickten Enes 10 des Stiletts 2 ist. Der Durchmesser der Öffnung 14 des Begrenzers 13 muß mit dem Kaliber des Endstückes des Injektionssystems übereinstimmen. Soll in der Hülse eine Spritze untergebracht werden, so ist der Durchmesser des Gehäuses 15 des Hülsengriffes 5 weit, dem Kaliber der Spritze entsprechend. In diesem Falle gibt es einen Begrenzer 13 und einen engeren Kanal 16, in den das verdickte Ende 10 des Stiletts 2 eingesetzt wird. Bei der Verwendung eines anderen Injektionssystems, zum Beispiel einer Gummiröhre mit einem Endstück, ist das Vorhandensein vom Begrenzer 13 nicht unbedingt. In den beiden Fällen wird zweckmäßigerweise der Hülsengriff 5 aus mechanisch festem durchsichtigem Kunststoff hergestellt. Dadurch wird die Zuverlässigkeit der Anwendung des Trokars vergrößert, das Anwendungsgebiet desselben, insbesondere für intravenöse und intrarterielle Infusionen durch Katheter wird erweitert, was sowohl in der medizinischen Praxis als auch in der Tierheilkunde von Bedeutung ist.

Der erfindungsgemäße Trokar arbeitet wie folgt. Nach einem kleinen Einschnitt in die Haut, der unter örtlicher Anästhesie gemacht wird, wird das spitze Ende 3 des Trokars in die Wunde unter einem Winkel gestellt, der von dem Anwendungsfall abhängt. Zum Durchstechen einer Brust- oder Bauchwand wird das Instrument zur Leibesfläche normal oder unter einem kleinen Winkel gestellt. Zur Implantation von Geweben in die Unterhaut oder intramuskulär hängt der Einstellwinkel des Instrumentes von der vermutlichen Tiefe des Endproduktes dessen Durchführung, von der Implantationsstelle usw. ab. Danach wird die Anästhesielösung durch das Drücken auf den Kolben der Spritze 6, die in dem Hülsengriff 5 des Trokars untergebracht ist, oder ein anderes Injektionsverfahren abgelassen. Das schafft vor dem abgespitzten Ende 3 des Stiletts 2 eine Betäubungszone und eine Zone der hydraulischen Präparierung von Geweben. Gleichzeitig oder ein wenig später schiebt man das Instrument durch das Drücken auf den Hülsengriff 5 im Gewebe vor.

Beim Durchstich einer Brust-, Bauchwand oder einer Harnblase für die Entleerung der Höhle von dem Inhalt, wenn die Höhlenwand durchgegangen ist, nimmt man das Stilett zusammen mit dem Hülsengriff 5 heraus, wobei ein freier Abfluß der Flüssigkeit erfolgt. Gegebenenfalls wird diese Flüssigkeit mit der Spritze 6 abgesaugt, wobei das Stilett 2 entfernt oder nicht entfernt ist. Wenn die Einführung von Heilmitteln erforderlich ist, so kann diese Manipulation durch die Spritze 6 oder ein anderes Injektionssystem verwirklicht werden, ohne daß das Instrument ausgebaut wird. Im letzteren Fall tritt die Flüssigkeit durch den Kanal 4 des Stiletts 2 und im anderen Fall - durch die Lichtweite der Hülse 1.

Ähnlicherweise erfolgt der Durchstich eines Epi- und Subduralraumes, das Absaugen der Rückenmarkflüssigkeit und die Einführung von Heilmitteln. Bei der Anwendung des erfindungsgemäßen Trokars zur Einführung des Katheters in eine Höhle, ein Blutgefäß usw., darunter auch in der Tierheilkunde, und zur Einführung eines Endoskoprohres nimmt man das Stillet 2 mit dem Hülsengriff 5 heraus, und durch die an der Stelle gebliebene Hülse 1 werden jeweils der Katheter oder das Endoskoprohr ein- oder durchgeleitet. Bei der Implantation einer Zellsuspension oder von Gewebestücken, nach dem Durchstechen oder Tunnelieren der Unterhaut, der Muskel nimmt man das Stilett 2 mit dem Hülsengriff 5 heraus und führt durch die Hülse 1 das zu implantierende Material ein.

## Gewerbliche Anwendbarkeit

Der erfindungsgemäße Trokar kann entweder als metallines Instrument für die mehrmalige Anwendung oder einmaliges Instrument, gegossen aus harten Kunststoffen, zweckmäßigerweise mit einem durchsichtigen Hülsengriff 5 oder einer als Griff dienenden Spritze ausgeführt werden.

Die industrielle Fertigung ist einfach und die Anwendung erfordert keine Neuausbildung von medizinischem Personal.

Der erfindungsgemäße Trokar kann auf dem Gebiet der Medizin und Tierheilkunde in allen Fällen verwendet werden, wo man Punktionskanülen und herkömmliche Trokare benutzt. Außerdem kann er zur Einführung von Endoskopen in verschiedene Leibeshöhlen durch die Haut, zur Implantation von Zellsuspensionen und Zellkulturen sowie Organ- und Gewebestücken verwendet werden.

Einer der Hauptvorteile des erfindungsgemäßen Trokars ist die Verminderung des Traumatismus und Erhöhung der Sicherheit seiner Anwendung. Es wird durch die Form des Schneidteils des Instrumentes und die Möglichkeit der gleichzeitigen Einführung einer Lösung für die hydraulische Präparation der Gewebe vor den Schneiden erzielt. Dasselbe ermöglicht es, Höhlen zu punktieren, die keine Flüssigkeit enthalten, wodurch das Anwendungsgebiet des Instrumentes erweitert wird, die Laparotomie durch die Laparozentese ersetzt werden kann, wobei die Hospitalisierungsdauer, insbesondere bei einer Endoskopie und Implantation von Gewebe- und Organstücken gekürzt wird.

Der Aufbau des Trokars, und zwar eine mehrflächige Form des Schneidteils, die Möglichkeit der Auswahl des Schneidenwinkels, des Durchmessers des Stiletts und der Hülse erleichtert auch dessen Durchgehen durch die Gewebe verschiedener Dichte.

Ein wesentlicher Vorteil des Trokars ist die vollständige Anästhesierung von mit dessen Hilfe ausgeführten Manipulationen durch Vereinigung der Anästhesie mit dem Vorschub des Instrumentes in Geweben.

PATENTANSPRÜCHE

1. Trokar, enthaltend ein Stilett (2), das ein abgespitztes Ende (3) aufweist, und eine Hülse (1), in der das Stilett (2) angeordnet ist, dadurch g e k e n n z e i c h n e t, daß das Stilett einen durchgehenden Längskanal (4) und dessen abgespitztes Ende mindestens drei Flächen hat.

2. Trokar nach Anspruch 1, dadurch g e k e n n z e i c h n e t, daß der Schärfungswinkel der Schneidflächen des abgespitzten Endes (3) des Stiletts (2) zu seiner Achse von 45 bis 90° beträgt.

3. Trokar nach Anspruch 1, 2, dadurch g e - k e n n z e i c h n e t, daß die Schneidflächen Schneidkanten aufweisen.

4. Trokar nach Anspruch 1,2,3, dadurch g e - k e n n z e i c h n e t, daß das eine Ende des Kanals (4) mindestens einen Austritt (11) in unmittelbarer Nähe der Spitze zwischen den benachbarten Flächen hat, und das andere Ende des Kanals (4) in Übereinstimmung mit den geometrischen Maßen von in diesen einzusetzenden Injektionssystemen (6) erweitert ausgeführt ist.

5. Trokar nach jedem beliebigen Anspruch, dadurch g e k e n n z e i c h n e t, daß er mit einem abnehmbaren Hülsengriff (5) versehen ist, dessen geometrische Maße den geometrischen Maßen des Behälters von Injektionssystemen (6) entspricht.

FIG. 1

FIG. 2

FIG. 3          FIG. 4

# INTERNATIONAL SEARCH REPORT

0346469

International Application No PCT/SU 87/00130

## I. CLASSIFICATION OF SUBJECT MATTER (if several classification symbols apply, indicate all)

According to International Patent Classification (IPC) or to both National Classification and IPC

IPC$^4$   A61B 17/34

## II. FIELDS SEARCHED

### Minimum Documentation Searched [7]

| Classification System | Classification Symbols |
|---|---|
| IPC$^4$ | A61B 10/00, 17/34 |

Documentation Searched other than Minimum Documentation
to the Extent that such Documents are Included in the Fields Searched [8]

## III. DOCUMENTS CONSIDERED TO BE RELEVANT

| Category [*] | Citation of Document, [11] with indication, where appropriate, of the relevant passages [12] | Relevant to Claim No. [13] |
|---|---|---|
| A | EP, A2, 0120701 (CODMAN & SHURTIEFF INC.) 3 October 1984 (03.10.84) see page 7, lines 17-18, figure 1 | 1-5 |
| A | FR, A1, 2289156 (DOW CORNING CORPORATION) 28 May 1976 (28.05.76) see page 2, lines 35-40, figure 2 | 1-5 |
| A | GB, A, 1149849 (BRUNSWICK CORPORATION) 23 April 1969 (23.04.69) see page 2, lines 4-9, figure 4 | 1-5 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 20 May 1988 (20.05.88) | 8 August 1988 (08.08.88) |

| International Searching Authority | Signature of Authorized Officer |
|---|---|
| ISA/SU | |

Form PCT/ISA/210 (second sheet) (January 1985)